# EUROPEAN PATENT SPECIFICATION

(11) **EP 1 596 826 B1**
(45) Date of publication and mention of the grant of the patent: **28.04.2010**
(21) Application number: 04710372.6
(22) Date of filing: 12.02.2004
(51) Int. Cl.: A61K 9/127, A61K 31/79

(54) **USE OF PVP-IODINE LIPOSOMES FOR TREATMENT OF ATOPIC DERMATITIS**
VERWENDUNG VON PVP-IOD LIPOSOMEN ZUR BEHANDLUNG VON ATOPISCHER DERMATITIS
UTILISATION DE LIPOSOMES DE POLYVIDONE IODEE POUR LE TRAITEMENT DE LA DERMATITE ATOPIQUE

(30) Priority: 24.02.2003 EP 03003283
(43) Date of publication of application: 23.11.2005
(73) Proprietor: EURO-CELTIQUE S.A., 1653 Luxembourg (LU)
(72) Inventor: REIMER, Karen, 65582 Hambach (DE); FLEISCHER, Wolfgang, 55218 Ingelheim (DE)
(74) Representative: Glas, Holger
(86) International application number: PCT/EP2004/001317
(87) International publication number: WO 2004/073683

(56) References cited:
- WO-A-00/72822

## Description

The present invention concerns the use of PVP-iodine liposomes for treatment of atopic dermatitis. The present invention also concerns the use of a pharmaceutical preparation for the treatment of atopic dermatitis, wherein the preparation comprises at least one antiseptic compound in a pharmaceutically effective amount combined a particulate, pharmaceutically acceptable carrier.

Atopic dermatitis, also known as neurodermatitis, atopic oder endogeneous eczema, is permanent or recurring itchy inflammation of the skin accompanied by the formation of blisters, nodules and lapidosis. Symptom-free periods are usually repeatedly interrupted by malady outbreaks of varying severity. Due to the permanently dry and often itchy skin, affected persons experience atopic dermatitis as a permanent psychological stress. Patients have moreover an increased susceptibility to other atopic ailments such as asthma or maladies caused by hypersensitivity such as allergies of the nose (rhinitis), of the eyes (conjunctivitis) or the ears (otitis).

Data on the frequency of atopic dermatitis varies between 10% and 20% for children and between and 3% and 5% for adults. It is however clear that the incidence of the ailment has been continuously increasing over the last few years.

The afore-mentioned general symptoms can be divided into different stages according to the age of the afflicted patient.

The clinical phenotype of atopic dermatitis is generally characterized by the onset, mostly during early childhood, of itching, redness, desquamation, wet and crust formation of the skin predominantly on the cheeks and scalp (milk crust). During childhood mostly the opposite part of the joint flexures are generally first afflicted. Later the joint flexures themselves, frequently also the backside, are afflicted with atopic dermatitis. In adulthood, the face, throat, neck, shoulders and breast as well as the flexures are the most common sites of skin alterations. A white dermographism can be triggered.

Frequently, atopic dermatitis is characterized by bacterial and virus secondary infections. An increase of the IgE concentration and eosinophiles in the blood can be observed.

The different forms of atopic dermatitis during childhood and adulthood have a dysfunction of the sebaceous and sweat glands in common. This leads to a dull and dry skin with an enlarged surface structure (so-called lichenification). As a result of constant scratching nails are mostly worn and glossy and eyebrows become abraded due to the associated conjunctivitis.

Atopic dermatitis often appears in babies as a red exanthema (eczema) on the cheeks and as so-called milk crust on the scalp. The eczema then spreads to the face, throat and nappy region.

During infancy the inside of the arms and the back of the legs can also be affected. The eczema is usually dry. If it is contaminated with bacteria, a secondary infection (impetigonisation) can occur. The result of this bacterial secondary infection is that the eczema secretes fluid.

From the age of one or two onwards, the appearance of the malady changes. The eczema occurs predominantly at the arm and knee flexures as well as on the wrist and ankle joints. The throat may also be affected. The eczema can be very itchy and the skin may thicken due to repeated scratching.

In older children and adults the eczema can occur over the whole body. Frequently, the face and the flexures of arms and legs are afflicted. However, the upper part of the body and the throat may also be afflicted. The skin is dry, blotchy, reddened and thickened. Bacteria can easily cause the eczema to become inflamed.

Many people afflicted with atopic ailments also display so-called atopic stigmata. That may take the form of dark-ringed eyes, furrows below the eyes, cracks in the earlobes and itching due to sweating or direct contact with wool.

The causes of atopic dermatitis are largely unknown. It is assumed that a genetic predisposition exists. Additional factors include immunological reactions against allergens as well as a disorder of the immune system. It is suspected that there is a strong auto-immunological component connected with atopic dermatitis (see e.g. IgE increase, eosinophilie). However, other triggering factors comprise non-immunological factors, such as predominantly psychovegetative and neurovegetative disorders, as well as disturbances of skin metabolism. Concerning the genetic predisposition it is assumed that it is polygenic.

The various designations for atopic dermatitis which, as mentioned above, is also known as neurodermatitis, atopic or endogenic eczema, depend on its appearance and where it appears. It may be, e.g. eczema flexurarum, that afflicts the joint flexures, pyodermas due to bacterial and viral secondary infections such as eczema molluscatum or eczema verrucatum, eczema dyscoides, dyshidrotic, microbial, nummular or seborrhoic eczema. The pyodermas may also comprise e.g. *Impetigo contagiosa* and its derivatives as well as *Folliculitis barbae*.

The known therapies for the treatment of atopic dermatitis and the other afore-mentioned eczemas comprise different strategies.

In cases of acute attacks of atopic dermatitis that may, e.g. by triggered by passive smoking, stress, emotional stress, food such as milk and citrus fruits, allergens such as mites or pollen, clothing or climatic influences, creams comprising corticoids such as cortisone and/or urea are generally applied. Cortisone has an anti-inflammatory effect, while urea reduces itching and increases skin moisture. Other therapies for atopic dermatitis include e.g. body exposure to UV-A und UV-B after salt water baths, oral intake of antihistamines, immuno-modulators such as Cyclosporin A und Interferon-γ or topical treatment with tacrolimus.

A drawback of cortisone-containing creams is that skin atrophy can occur after long treatment. Due to the systemic uptake and immuno-suppressive action, an increasing sensitivity towards compounds of allergenic potential can also occur. These include among others the metabolic products of fungae occurring in healthy natural skin. For this reason, attacks which occur shortly after cortisone treatment has ceased are therefore often more intense than previous attacks. Special consideration has to be taken if children are to be treated with cortisone, since they have by nature a thinner skin. Moreover, periorbital dermatitis, which is a contact dermatitis, may occur as a side-effect after treatment with cortisone.

Even daily skin care with fatty oil baths, creams and lotions for the treatment of dry skin may be problematic, since contact allergies may arise due to their ingredients. Creams, ointments and lotions for the treatment of atopic dermatitis have therefore to be strictly controlled with respect to their composition and ingredients and should not contain e.g. common but unsuitable emulgators. Their production may be consequently more expensive.

Components which are common today include some ingredients which are actually contraindicated for people suffering from neurodermatitis and those suffering generally from dry skin: Mineral oils (the most frequent ingredient after water) and related substances such as Vaseline and waxes may leave the skin feeling pleasantly smooth, but in the long run they do however impede the regenerative forces of the skin. This also applies to silicone oils which have become widespread by now. Additionally, the perfumes and preservatives frequently contained in the aforementioned compositions can be problematic for people suffering from neurodermatitis, since they may penetrate the skin extremely easily due to the damaged state of the skin.

The afore-mentioned compounds and formulations have moreover the drawback that while they have an anti-inflammatory and/or anti-itching effect, they do not allow for an efficient healing of the skin lesions resulting from the atopic dermatitis such as desquamation, crust formation and cracking of the skin.

Another drawback of the administration of hormones for the treatment of atopic dermatitis is that the andrenal cortex reduces its production due to the permanent external supply with hormones. Systemic deficiency syndromes (e.g. such as growth retardation) may occur.

The use of anti-bacterial or anti-viral compounds to combat bacterial and viral secondary infections, which occur in the course of atopic dermatitis, is also problematic. Especially the use of common antibiotics and antiseptics such as chlorohexidine, octenidine, neomycine and gentamcyine has e.g. the drawback that these compounds impede granulation and epithelisation of the skin, thereby substantially inhibiting the healing process (Kramer et al. "Toxikologische Anforderungen an lokale Antiinfektiva", in Kramer A, Wendt M, Werner M (eds): "Möglichkeiten und Perspektiven der klinischen Antiseptik". Wiesbaden, mhp, 1995, 35-40, Kramer et al., "Wundantiseptik" in Kramer A, Gröschel D, Hingst V, Lippert H, Rotter M, Weufen W (eds): "Klinische Antiseptik". Berlin, Springer, 1993, 163-191; Kramer et al., "Indikationen und Auswahlkriterien für lokale Wundantiinfektiva und Wundauflagen im Rahmen der chirurgischen Wundbehandlung" in Beck EG; Eikmann T, Tilkes F (eds): "Hygiene in Krankenhaus und Praxis", ecomed, 1999, 1-16).

The prior art also describes the use of PVP-iodine-containing solutions for the treatment of bacterial secondary infections in the course of atopic dermatitis. The use of antiseptics and particularly halogen-releasing antiseptics such as povidone-iodine, also known as polyvidone-iodine or PVP-iodine, e.g. poly(1-vinyl-pyrrolidine-2-on)-iodine complex, should have the general advantage that the development of resistances is avoided while retaining at the same time a high antimicrobial efficacy. Moreover, these antiseptic compounds rarely dispose allergenic properties compared to antibiotics.

In particular, a 10% povidone-iodine solution has been used to reduce the infectious titer of *Staphylococcus aureus* occurring during bacterial secondary infections in the course of atopic dermatitis (Akiyama et al. 1997, J. Dermatol. Sci. 16,23-30). However, this prior art shows that *Staphylococcus aureus* bacteria, which have already been in human plasma for more than 24 hours, can form biofilm-like structures that allow them to resist treatment with a 10% povidone-iodine solution.

Sugimoto et al. ((2002) Dermatology, 204 (suppl. 1), 63-69, have also studied the effect of povidone-iodine solution for the treatment of atopic dermatitis. An evaluation of the effect of povidone-iodine solution is however difficult in view of the fact that a steroid-containing ointment as well as a moisturizing ointment have been used for treatment of exactly those skin areas that showed the most severe damage.

The use of PVP-iodine containing solutions can have the additional drawback that the treated skin may show coloured patches as the solution may have a brown colour. Even though the colour may be removed by thorough washing, this is not acceptable where sensitive and already pre-damaged skin areas are to be treated.

In the treatment of skin damage as it appears in the course of atopic dermatitis, accessibility of the surface of the damaged skin parts is of essential importance for the effectiveness of the compounds used for treatment. One prior art document, namely the publication of Taylor et al. ((1987) Journal of Hospital Infection, 9, 22-29) deals with the efficacy of PVP-iodine solutions in test systems, where especially the anti-viral activity of antiseptic substances on surfaces is tested. Interestingly, this prior art document shows that, compared to other antiseptic solutions such as e.g. hypochloride solutions or alkaline glutaraldehyde, PVP-iodine solutions are significantly less effective in killing viral infectious agents that have been immobilized and dehydrated on surfaces.

Van Ketel et al. ((1990), Dermatol. Clin., 8, 107-109) have shown that the use of povidone-iodine-containing solutions, commercially available under the name Betadine (in the US) or Betaisadona (in Europe), may lead to allergic reactions and can thus be problematic.

It has also been found that common PVP-iodine ointments have a drying effect on the skin and are therefore contraindicated for wound healing as well as for the treatment of atopic dermatitis (Steen, (1993), Postgrad Med J, 69 (suppl. 3), 84-92). None of the prior art documents mentioned above mention the production of liposomes containing PVP-iodine for the treatment of atopic dermatitis.

The use of antiseptics and/or substances that promote wound healing for external application on humans and animals is disclosed in EP 0 639 373. In particular, this document describes liposome preparations of PVP-iodine for topical application to the external parts of the eye mainly for purposes of complete disinfection. These preparations generally take the form of a cream, an ointment, a lotion, a gel or drops.

Liposomes are well-known drug or compound carriers, so that administering medication in liposomal form has been the subject of investigation for quite some time. An overview of the administration of compounds in liposomal form is provided by the review "Targeted delivery to the pilosebaceous unit via liposomes" (Lauer et al. (1996) Advanced drug delivery reviews, 18, 311-324). The physical-chemical characterization of liposomal preparations at their therapeutic applications for the treatment of the pilosebaceous unit are described therein. Compounds administered via liposomes that have been investigated include e.g. anti-cancer agents, peptides, enzymes, anti-asthmatic and anti-allergic compounds and, as mentioned above, also antibodies. Liposomes or other carriers are not known as carriers of antiseptic compounds for the treatment of atopic dermatitis.

Looking at the afore-mentioned prior art it becomes clear that there is a need for well-tolerated, easily applicable pharmaceutical preparations for the treatment of atopic dermatitis as well as of the bacterial and viral secondary infections that frequently go with it. In particular, there is a need for topically applicable pharmaceutical preparations that are basically preferable over preparations that are to be administered systemically.

One objective of the present invention is to provide for a well-tolerated, easily applied pharmaceutical preparation for the topical treatment of atopic dermatitis that allows for lasting, efficient and scar-free treatment of the symptoms of even severe forms of atopic dermatitis as well as of the bacterial and viral secondary infections that frequently accompany it. These and other objectives of the present invention, as will become clear from the description, are solved by the subject-matter of the independent claim. Preferred embodiments of the invention are defined by the dependent claims.

The object of the present invention concerns the use of iodine or at least one iodine complex, which contains the iodine in elementary form, for the manufacture of a pharmaceutical preparation for the treatment of substantially all forms of atopic dermatitis, the preparation comprising the compound(s) in a pharmaceutically effective amount combined with pharmaceutically acceptable liposomes.

It has been surprisingly found that pharmaceutical preparations according to the invention that comprise an antiseptic compound such as PVP-iodine associated with particles such as liposomes are ideally suited for topic treatment of the various forms of atopic dermatitis as well as of the bacterial and viral secondary infections that frequently accompany it. It was particularly surprising that the inventive use of PVP-iodine-containing liposomes for the treatment of atopic dermatitis leads to a significant reduction of damaged skin parts and thus to a reduction of inflammation as well as itching, even when no bacterial or viral secondary infections are present.

According to the invention, the novel use of particle-containing preparations comprising antiseptic compounds such as PVP-iodine for the treatment of the various forms of atopic dermatitis has moreover the surprising advantage that fast and scar-free healing of the skin damage or lesions caused by atopic dermatitis is possible.

Moreover, the use according to the invention of PVP-iodine-containing particulate carriers such as e.g. liposomes in the form of e.g. an ointment, a lotion or a gel has an anti-inflammatory and/or itch-relieving effect.

Thus, use according to the invention of PVP-iodine-containing liposomes allows for the efficient treatment of various forms of atopic dermatitis wherein the topical application of a pharmaceutical preparation enables the efficient treatment of the underlying causes as well as of the symptoms of the various forms of atopic dermatitis. The use according to the invention of PVP-iodine-containing liposomes also has the advantage that the liposomes facilitate a faster healing of skin damage occurring as a result of atopic dermatitis without the side effects that previously established preparations elicited. Where bacterial secondary infections are treated by the use of PVP-iodine-containing liposomes, another advantage is that the existence of primary resistances and the development of secondary resistances of the infecting bacteria do not jeopardize successful treatment, as is the case when antibiotics are used.

Since the antiseptic compounds are formulated with particulate carriers, preferably liposomes, use according to the invention of PVP-iodine-containing liposomes has the additional advantage that the active compounds can reach deeper skin layers due to the liposomal formulation, as compared to other common ointment, gel and lotion formulations. Thus, an efficient topical application, which guarantees minimal side effects due to the restricted local use of the PVP-iodine-containing liposomes, is possible. Moreover, the use according to the invention of PVP-iodine liposomes promotes granulation and epithelisation of the skin and thus wound-healing.

The invention is thus based on the surprising finding that liposomes, are exceptionally well-suited as carriers for a iodine or at least one iodine complex, which contains the iodine in elementary form, especially for PVP-iodine for the application and treatment of mild and severe forms of atopic dermatitis and other forms of dermatitis mentioned above.

The preparations according to this invention permit protracted release of the compound(s) and render possible a lasting and local activity at the desired spot by interacting with the respective skin cell surfaces. Without wanting to by bound to a specific scientific theory, it is assumed that the outstanding effect of the PVP-iodine liposomes according to the invention is due to the deeper penetration of the liposomes into the damaged skin areas compared to conventional preparations. In this way, the compound(s) is/are transported more efficiently to the damaged skin parts. However, it is then surprising that such a radically effective substance class such as antiseptics does not impinge on the healing process of the especially sensitive and damaged tissue and can even suppress the formation of scar tissue, neoplasms, intergrowth etc.. This may be due to the granulation and epithelium-forming effect of the liposomal preparations.

In the context of the present invention, the term "atopic dermatitis" comprises those skin ailments that are due among other things to genetic predisposition and can also be triggered by the interplay of multiple immunological and non-immunological factors. Immunological factors comprise reactions against environmental allergens as well as auto-immunological reactions. Non-immunological factors include a number of emotional and neuro-vegetative disorders such as e.g. stress and metabolic disorders of the skin. Further factors include the circumstances mentioned above, which frequently cause acute attacks of the atopic dermatitis.

According to the invention the term "atopic dermatitis" is equivalent to the clinical pictures termed "neurodermatitis", "atopic eczema" or "endogeneous eczema". Particular forms of atopic dermatitis, which get their names from the place where they occur or from their appearance or from the stress factors which provoke them, are, according to the invention also comprised by the term "atopic dermatitis". These include in particular Eczema flexurarum, Eczema mulluscatum, Eczema verrucatum, Eczema vaccinatum, Eczema dyskoides, dyshydrotic eczema, microbial eczema, nummular eczema, seborrhobic eczema and other forms of eczema. Additional clinical pictures falling under the term atopic dermatitis are the perioral dermatitis and the periorbital dermatitis. According to the invention the term atopic dermatitis especially comprises the frequently occurring bacterial secondary infections such as those due to e.g. *Staphylococcus aureus* infections, pyodermas such as *Impetigo contagiosa* and its derivatives as well as the *Follicularis barbae* or viral secondary infections.

In the context of the present invention, antiseptic compounds first and foremost comprise compounds designated as antiseptics and used as such in the prior art. According to the invention these compounds especially comprise such disinfecting compounds that are pharmaceutically acceptable and can be used for the treatment of the various forms of atopic dermatitis mentioned above by topical application, as long as they have a formulation according to the invention. The antiseptic compounds preferably comprise, among other things, oxygen-releasing or elementary halogene-releasing compounds as well as metal compounds, such as silver and mercury compounds. More preferably, the antiseptic compounds according to the invention comprise halogene-releasing compounds such as iodine, iodine complexes and PVP-iodine which contain the iodine in elementary form.

In the context of the present invention the term "pharmaceutically effective amount" refers to an amount of the iodine or iodine complex in the preparations that is sufficient for treating the various afore-mentioned atopic dermatitis forms efficiently.

According to the invention, by using the preparations according to the invention, the various forms of atopic dermatitis can be treated topically in such a way that the damage resulting from the atopic dermatitis such as cracks, craters, reddening, crust-formation and erosions heal in effect completely, i.e. scar-free. Itchiness is also significantly reduced.

This effect is due to the surprising and unexpected fact that by using the preparations according to the invention such as PVP-iodine-containing liposomes, hyperkeratosis and uncontrolled growth of tissue can be avoided. Serious functional and cosmetic skin damage threatened by uncontrolled growth during the formation of new skin tissue can thus be avoided. Scar-free healing of the atopic dermatitis is highly relevant from a cosmetic viewpoint, since the atopic dermatitis which affects the face as well as the itchiness are usually experienced by the victim as extremely unpleasant. The fact that the use of e.g. PVP-iodine-containing liposomes allows for such efficient treatment of the various forms of atopic dermatitis was especially surprising, since it could not be expected that by using preparations comprising only one active compound the various reasons underlying atopic dermatitis (such as the immunological reaction, reduced sebum production as well as the bacterial and viral infections and inflammations) can be treated simultaneously and efficiently by topical application without scarring occurring or remaining.

PVP-iodine liposomes according to the invention ensure moreover that the damaged skin parts remain germ-free and also have sufficient moisture to ensure efficient healing.

PVP-iodine liposomes according to the present invention may therefore also be used for cosmetic purposes.

By use of preparations according to the invention the formation of scar tissue on the skin can be reduced and hyperkeratosis can be completely repressed. Intergrowth or the formation of neoplasms, that can lead to scars, are also significantly reduced upon use of e.g. PVP-iodine-containing liposomes for the treatment of the various forms of atopic dermatitis. Moreover, the use of the afore-mentioned preparations for the treatment of atopic dermatitis also allows for efficient killing of the bacterial infections that often make a major contribution to atopic dermatitis. Thus, antibiotics do not have to be administered as a supplementary therapy. The danger of resistance development does accordingly also not exist.

The composition of the liposomes, the concentration of the active compound(s) and methods for producing the PVP-iodine liposomes or preparations comprising an antiseptic compound in a pharmaceutically effective amount combined with the particulate carriers are presented below. If, for purposes of demonstration, liposomes and PVP-iodine are mentioned, the person skilled in the art is well aware that other carriers and other antiseptics may be formulated in analogous manner and may thus also be used for the same purposes.

The preparations according to the invention may also be used for treating the symptoms of the various forms of atopic dematitis such that a cosmetically acceptable and satisfying result is achieved for the affected persons. This aspect of the invention may also be designated as cosmetic remodelling.

Preparations according to the invention whose use permits the efficient treatment of the various forms of atopic dematitis can be produced by loading liposomes with PVP-iodine according to methods known in the art. The nature or composition of the liposomes is generally not decisive for the treatment success and can vary. The liposomal preparation, as for example described in EP 0 639 373, can be administered in different forms including, e.g. an ointment, a cream, a spray, a lotion, a solution, a suspension, a dispersion or a gel.

Preferably, the liposome-forming material is selected so that it does not react substantially or hardly reacts at all with the antiseptic compounds. One will therefore try to keep the content of chemically reactive substances as low as possible, if the antiseptic might be a reaction partner. If the antiseptic can release oxygen or halogen atoms, as is the case with PVP-iodine, one will use e.g. cholesterol which has a reactive double bond only in small amounts, if at all. In any case, one will choose the amount of such potentially reactive liposome-forming substances such that the pharmaceutical preparations have a sufficient storage stability of at least one, or even of at least two years (in compliance with statutory regulations). The storage conditions may comprise a temperature range of approximately -20 °C to approximately 60 °C.

The preparations according to this invention often contain the active compound(s), such as PVP-iodine, encapsulated in the liposomes. However, it may also occur that there is an amount of compound not contained inside the carrier. The compound(s) may also be associated with the surface of the particle liposomes.

In one embodiment of the invention the major part or even the whole amount of the active compund(s) may be located outside the liposomes.

The preparations according to the invention then may show a marked initial effect which is observed in addition to the slower, protracted release of the active agent from the carrier. This effect is especially observed where the carrier comprises liposomes. Without wishing to be bound to any theoretical explanation, it is presently assumed that in addition to the active agent encapsulated inside the liposomes, some active agent is present outside of the liposomes, and probably loosely bound to the outer surfaces of the liposomes. This could be due to complex association of active compound molecules with the liposomal membrane, or it could be due to active compound molecules forming a layer on the liposomal surface, which layer partly or even fully coats the liposome externally. The type and amount of this initial compound effect can e. g. be influenced by choice of the concentration parameters.

In the context of the present invention, protracted or prolonged release means that the active compound(s) is/are released form the pharmaceutical preparation over a time period between 1 to 24 hours.

The association of antiseptic compounds with liposomes, i.e. that active compounds can be included in the interior of liposomes or, depending on the circumstances, can also associate with the surfaces, depends among other things on the components used for formation of the liposomes.

In a preferred embodiment, preparations according to the invention used for the treatment of the various forms of atopic dematitis can comprise, besides the antiseptic compound also other anti-inflammatory agents and agents promoting wound-healing.

These additional anti-inflammatory comprise e.g. phenolic compounds, detergents, alcohols, organic disinfectants including among other things formaldehye-releasing compounds, phenolic compounds including alkyl and aryl phenolic compounds, as well as halogenated phenolic compounds, chinolines, acridines, hexahydropyrimidines, quaternary ammonia compounds, iminium salts and guadinines. Agents promoting wound-healing comprise those substances that have been described in the literature for such applications. Such compounds comprise substances that are known for promoting the formation of epithelial tissue. These include vitamins, particularly from the vitamin B group, alantoin, some azulenes, etc.

In some embodiments of the present invention, preparations according to the present invention comprise antiseptic compounds, preferably PVP-iodine, and may also comprise compounds such as agents promoting wound-healing or anti-inflammatory compounds.

Inventive preparations can also contain other customary agents, including adjuvants and additives, antioxidants, conserving agents or consistency-forming agents such as viscosity-adjusting additives, emulgators, etc. The person skilled in the art will select these adjuvants and additives in such a way that the ability of preparations, substantially consisting of particulate carriers such as liposomes and antiseptic compounds such as PVP-iodine, to efficiently treat the various forms of atopic dematitis, is not impaired. Additives may also comprise salts that allow for the regeneration of the active compound, such as the released halogen atom in case of halogen-releasing compounds. In the case of PVP-iodine such an additive may be KIO₃. Other additives that mediate or enhance penetration of the liposomes into the skin may also be part of the inventive preparations. Such additives comprise e.g. DMSO.

The amphiphilic substances generally known in prior art to form liposome membranes can be employed in the context of the invention as long as they are pharmaceutically acceptable for the intended application. Presently, liposome-forming systems comprising lecithin are preferred. Such systems can comprise hydrogenated soy bean lecithin besides cholesterol (if suitable despite its reactivity) and disodium succinatehexahydrate. Preferably one will make sure that the liposome-forming materials do not show any reactivity with the antiseptics in order to ensure the required storage stability of commercial products. Due to its double-bond reactivity high cholesterol contents will be avoided where it is to be formulated in connection with oxygen-releasing or halogen-releasing antiseptics (such as PVP-iodine). It is presently specifically preferred to use hydrogenated soy bean lecithin as the sole membrane-forming agent. Commercially available products such as Phospholipon® 90 H (Aventis, Germany) or Lipoid S100-3 (Lipoid GmbH, Germany) are also preferred.

As can be taken from the review of Lauer A.C. et al. 1995 (*vide supra*) phospholipid-based liposomes may also be generally used for production of liposomes that discharge their cargo into the skin. According to this review, the use of non-ionic liposomes, which can be formed with phosphatidylcholin, is also an option. The presence of sebum in the hair follicle may be relevant for the choice of components that the liposomes are formed from. Other components that may be used for the formation of micelles are also known to the person skilled in the art and may be used for the production of preparations according to the invention.

The known prior art methods for forming liposome structures can generally be used in the context of the invention. Broadly, these methods comprise mechanical agitation of a suitable mixture containing the membrane-forming substance and water or an aqueous solution. Filtration through suitable membranes is preferred in order to form a substantially uniform liposome size.

The average size of the liposomes according to this invention can vary over a broad range, generally from about 1 nm to about 100 µm. Liposomes or particulate carriers having diameters in the range of about 1 µm and 70 µm are preferred. The person skilled in the art knows that the efficiency of liposomal penetration into the skin increases with decreasing diameter and that therefore liposomes having diameters of about 1 µm to 10 µm, of about 5 to 7 µm or about 5 µm may also be used. Generally the size of liposomes should be selected such that a good penetration into the skin is guaranteed. A particularly preferred embodiment of the invention therefore comprises liposomes having a diameter of between about 1 and 25 µm.

Liposomes in more fluid preparations may be generally more suited for treatment of bacterial infections, while liposomes in more gel-like formulations are generally better suited for treatment of viral infections. It seems that symptoms that are due to viral infections are preferably treated with preparations according to the invention that allow for longer contact times with the affected body areas. Symptoms due to bacterial infections may be treated preferably with preparations that have rather short contact times with the affected body areas.

Generally, liposomes having a rather small average diameter are better suited for production of solutions, dispersions, suspensions. Such rather small diameters typically comprise diameters of around 1 µm to 10 µm, or even smaller in the case of solutions. In contrast, gel or ointment formulations may comprise liposome of a size of about 1 µm to 50 µm.

According to the invention the use of inventive liposomal preparations is with the treatment of the different forms of varying severity of atopic dermatitis particularly if the inventive preparations comprise PVP-iodine and liposomes. The use of the inventive liposomal preparations for treatment of the various forms of atopic dermatitis, irrespective of the degree of severity, has the advantage that, as no antibiotics are used, no resistances develop thus leading to a reduction of allergic reactions.

Thus, the use of inventive preparations for topical treatment of different forms of atopic dermatitis allows for an efficient treatment of the various forms of atopic dermatitis with significantly less side effects and without the formation of scar tissue. A systemic application of the active components, as applied during the treatment of severe forms of atopic dermatitis with e.g. antibiotics or hormones, is not necessary. Correspondingly, unwanted side effects are reduced.

Another advantage of the inventive liposomal antiseptic preparations is that they are also active against viruses, which antibiotics are not. Such inflammatory reactions that also contribute to the atopic dermatitis phenotype, but which are not due to bacterial infections, can also be efficiently treated as part of an atopic dermatitis treatment with the inventive liposomal preparations. Moreover, a liposomal preparation with an antiseptic compound such as PVP-iodine allows for a protracted compound-release from the liposomes. This leads to prolonged release of the antimicrobial substance which allows for less frequent application as compared to the common antiseptic solutions. Due to the liposomal composition and the corresponding choice of additives with the afore-mentioned functions, it is ensured that the inventive use of the preparations does not lead to inflammatory and/or allergic skin reactions with patients suffering from atopic dermatitis. On the contrary such unwanted reactions are indeed avoided through the use of adequate liposomal components. That liposomes have moisturizing effect on damaged skin is another advantage of preparations according to the invention.

Preparations according to this invention can take a variety of forms, including pharmaceutically acceptable solid or liquid formulations such as emulsions, dispersions, suspensions, solutions, gels, ointments, waxes, spray, lotions, etc. The formulation as a Hydrogel is preferred. If in the context use is made of the term "gel", this thus always includes the preferred embodiment of a Hydrogel.

Generally, the amount of active agents in an inventive preparation will be determined by the desired effect on the one hand and the carrying capacity of the carrier preparation for the agent on the other hand. Broadly speaking, the amount of active agent in an inventive carrier preparation can range in concentrations between the lower limit of effectiveness of the agent and the maximum loading of the agent in the respective carrier preparation. It is understood that the active compounds are present in the inventive preparations in a pharmaceutically sufficient amount, i.e. in an amount sufficient for treatment of the different forms of atopic dematitis.

More specifically, for an antiseptic agent, such as povidone iodine, a solution, dispersion, oil , ointment or gel in an inventive carrier preparation, where the carrier is a liposome preparation, can contain between 0.1 and 10 g of agent in 100 g of preparation. Such a preparation will then typically contain between 1 and 5 g of liposome membrane-forming substance, especially lecithin, per 100 g of preparation.

Usually preferred active compound concentrations such as of e.g. PVP-iodine concentration are normally between 1% to 10% and preferably 1% to 5% by weight.

In a lotion, which can be a hydrophilic or a lipophilic lotion, a typical range of active compound such as PVP-iodine will be between 0.5 and 10 g compound, and between 1 and 5 g, preferrably about 4 g of liposome membrane forming agent such as hydrogenated soy bean lecithin per 100 g of lotion. In the case of a hydrophilic lotion, electrolyte solution will often be used in preparing the liposome-containing lotion.

A lipophilic lotion will often be made from compound, membrane-forming substance and lipophilic formation agents such as medium chain length triglycerides, etc.

A hydrophilic cream comprising an inventive liposome preparation will generally comprise between 0.1 and 10 g agent, such as PVP-iodine, together with between about 1 and 10 g membrane forming substance and further typical O/W cream forming additives per 100 g of cream.

A comparable amphiphilic cream according to the invention will have similar contents of agent and membrane forming substance such as lecithin, and will have the typical further additives of an amphiphilic cream.

A hydrophilic ointment according to the invention can broadly comprise between 0.1 and 10 g compound and between 1 and 10 g liposome membrane forming substance such as lecithin, together with typical prior art ointment basis substances such as Macrogol (TM) and water in 100 g of ointment.

A non-alcoholic hydrogel according to the invention could broadly comprise between 1 and 5 g compound such as PVP-iodine, approximately 2-4 g lecithin and gel-forming substances such as Carbopol (TM), with pH-adjusting agent and water to form 100 g of hydrogel.

An inventive aerosol or spray preparation will often comprise up to 50 mg, but could comprise up to and above 100 mg of liposomal active compound formulation per unit spray dose. The spray preparation will typically comprise at least 10 % wt of compound agent such as PVP-iodine in the loaded liposomes (or alternative carrier particles), but may comprise up to 50 % wt or even more of active agent. Where the active agent is PVP-iodine, the amount of available iodine will generally be about 10 % wt (based on PVP-iodine).

More specific formulations are notable from the embodiment examples.

The features and advantages of this invention will become clear in more detail from the ensuing description of preferred embodiments. In these embodiments, which include a best mode, povidone iodine is exemplified as an antiseptic agent and liposomes are chosen as the carrier. This should however not be construed as a restriction of this invention to antiseptic agents alone or, among antiseptic agents, to povidone iodine, and/or to liposomes as the carrier, although such preparations are specifically preferred. According to the invention other particulate carriers such as "large porous particles" or other micelles, nanoparticles, etc. can be formulated with PVP-iodine in order to produce preparations that allow for an efficient treatment of the various forms of atopic dematitis. Correspondingly, other halogen-releasing antiseptics can be formulated with liposomes into a preparation that also allows for the efficient topical treatment of atopic dematitis. One preferred method for producing the invention's liposomes can generally be described as follows:

The lipid membrane-forming components, e. g. lecithin, are dissolved in a suitable solvent such as chloroform or a 2: 1 mixture of methanol and chloroform and are filtered under sterile conditions. A lipid film is then produced on a sterile high surface substrate, such as glass beads, by controlled evaporation of the solvent. In some cases, it can be quite sufficient to form the film on the inner surface of the vessel used in evaporating the solvent without using a specific substrate to increase the surface.

An aqueous system is prepared from electrolyte components and the (one or more) active agents to be incorporated in the liposome preparation. Such an aqueous system can e. g. comprise 10 mmol/l sodium hydrogen phosphate and 0.9 % sodium chloride, at ph 7.4; the aqueous system will further comprise at least the desired amount of the active agent, which in the embodiment examples is povidone iodin. Often, the aqueous system will comprise an excess amount of agent or agents. The pH of the buffer solutions may be varied.

The following table discloses buffer solutions which may be used for preparation of liposomal dispersions.

| | pH 5 | pH 5 | pH 5 | pH 6 | pH 7.4 | pH 7.4 |
|---|---|---|---|---|---|---|
| Na₂HPO₄ | 0.0114 g | | 0.1436 g | | | |
| Na₂HPO₄ x 2H₂O | | 1.8334 g | | 2.2464 g 2.2464 g | 0.445 g 0.445 g | 0.89 g 0.89 g |
| KH₂PO₄ | 0.8970 g | | 0.7973 g | | | |
| Citric acid | | 0.9312 g | | 0.7085 g | | |
| 1 M HCl | | | | | pH adjust. | pH adjust. |
| Water, purified | ad 100 ml | ad 100 ml | ad 100 ml | ad 100 ml | ad 100 ml | ad 100 ml |

Preparations may then finally be adjusted with NaCl or Glycerol to an isotonic range (250-350 mOsmol/kg). Depending on the PVP-iodine concentration the salt strength may also be varied (as e.g. shown for buffer solutions with pH 7.4 in the above table).

The liposomes are generally formed by agitating said aqueous system in the presence of said film formed by the lipid components. At this stage, further additives can be added to improve liposome formation; e. g. sodium cholate. Liposome formation can also be influenced by mechanical action such as pressure filtration through e. g. polycarbonate membranes, or centrifuging. Generally, the raw liposome dispersion will be washed, e. g. with electrolyte solution as used in preparing the above-described solution of the active agent.

When liposomes with the required size distribution have been obtained and washed, they can be redispersed in an electrolyte solution as already described, often also comprising sugars such as saccharose or a suitable sugar substitute. The dispersion can be freeze-dried, and it can be lyophilysed. It can, prior to use, be reconstituted by addition of water and suitable mechanical agitation at the transition temperature of the lipid component, which for hydrogenated soy bean lecithin is e. g. 55 C.

In the following Examples, hydrogenated soy bean lecithin (EPIKURON (TM) 200 SH obtainable from Lukas Meyer, Germany or PHOSPOLIPON (TM) 90 H obtainable from Nattermann Phospholipid GmbH, Germany) was used. However, other pharmaceutically acceptable liposome membrane forming substances can be used instead, and the person skilled in the art will find it easy to select suitable alternative liposome forming systems from what is described in prior art.

The person skilled in the art is well aware that the liposomes-containing dispersion may comprise additional additives and adjuvants that can influence the appearance of the liposomal preparations. The inventive liposomal dispersion may contain e.g. colour pigments that ensure that the slightly yellow or brown colours that are due to PVP-iodine or released iodine are not visible. In the same manner, liposomes or the pharmaceutical liposome-containing preparations may comprise additives that influence the consistency and the smell of the preparations.

The person skilled in the art is well aware that the choice of these additives and adjuvants depends on the intended application form of the preparation (e.g. as ointment, gel or solution) and may be influenced by aesthetic considerations (such as colour and smell).

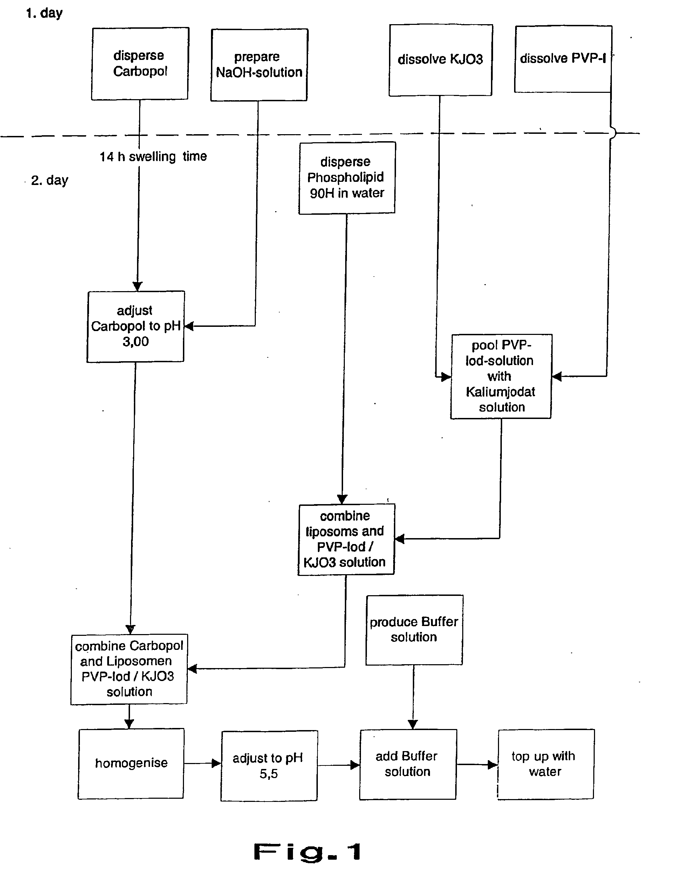

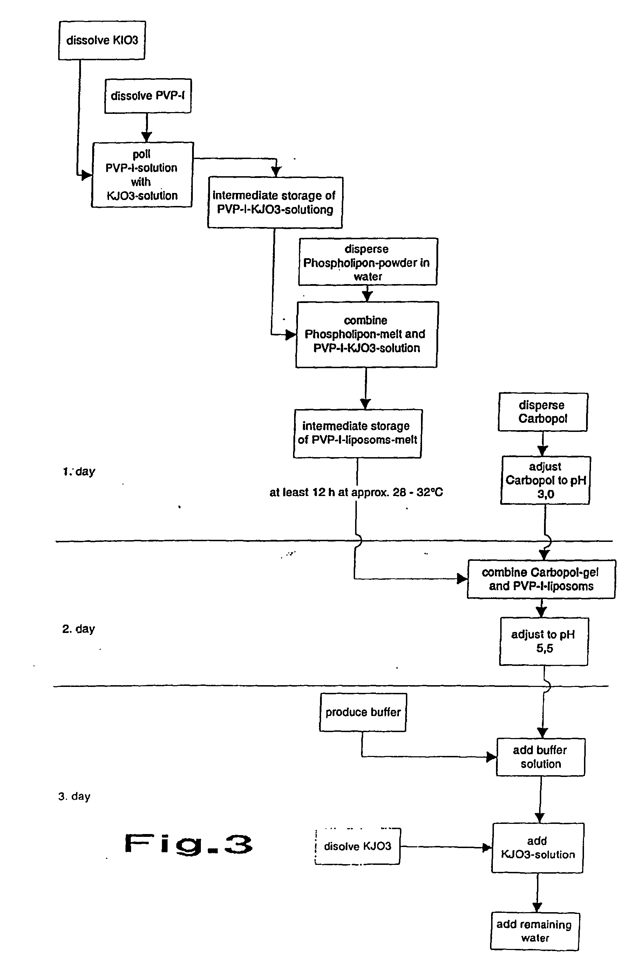

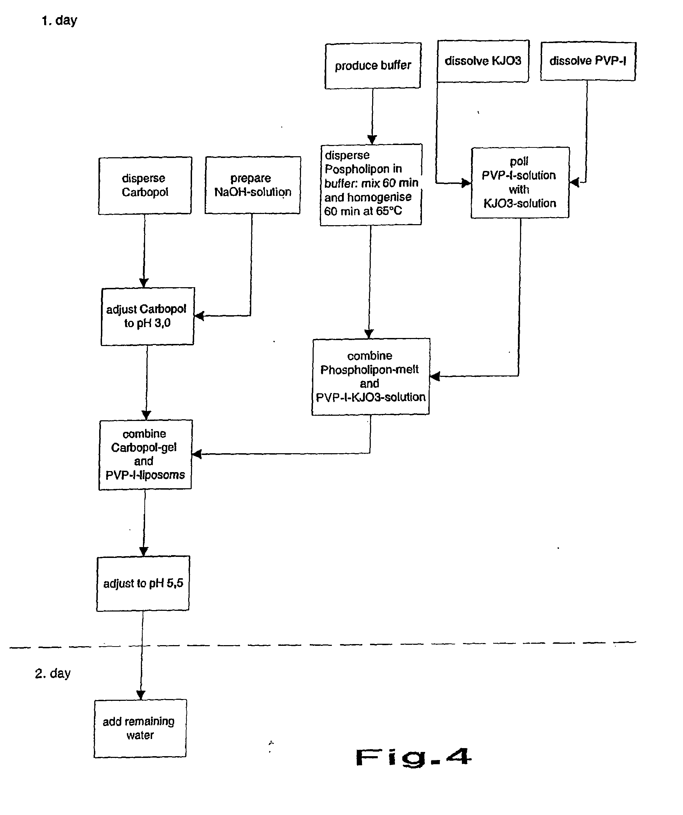

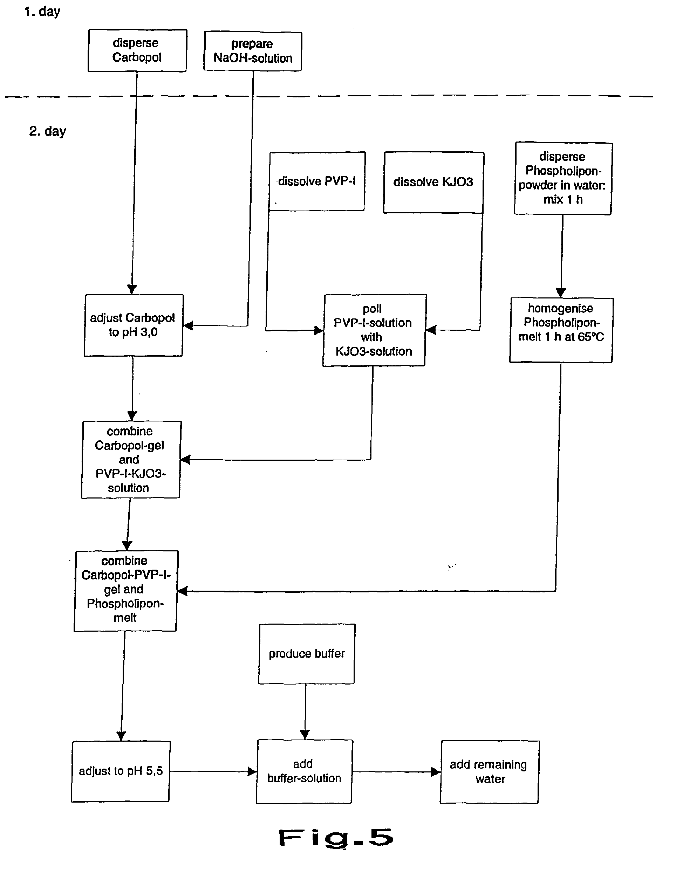

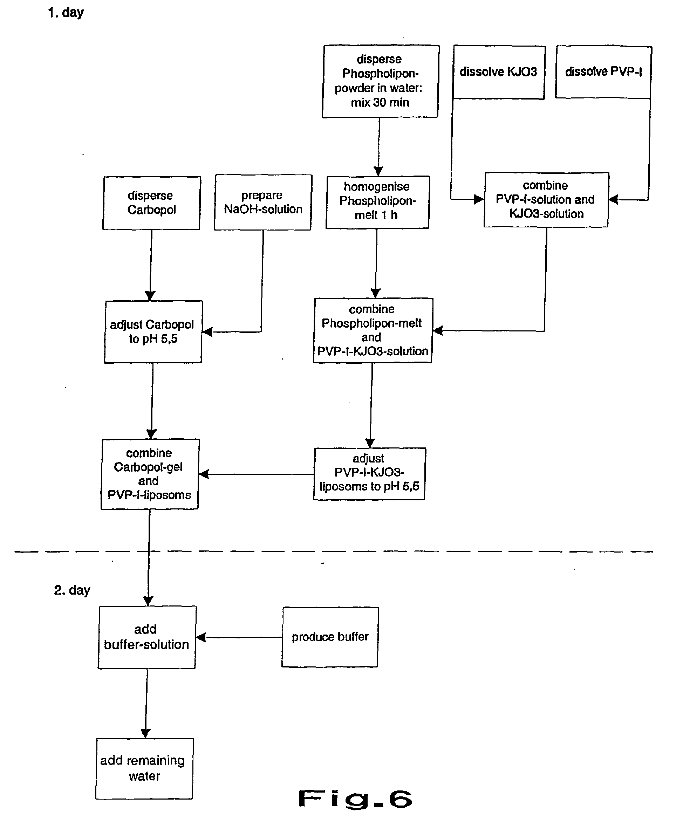

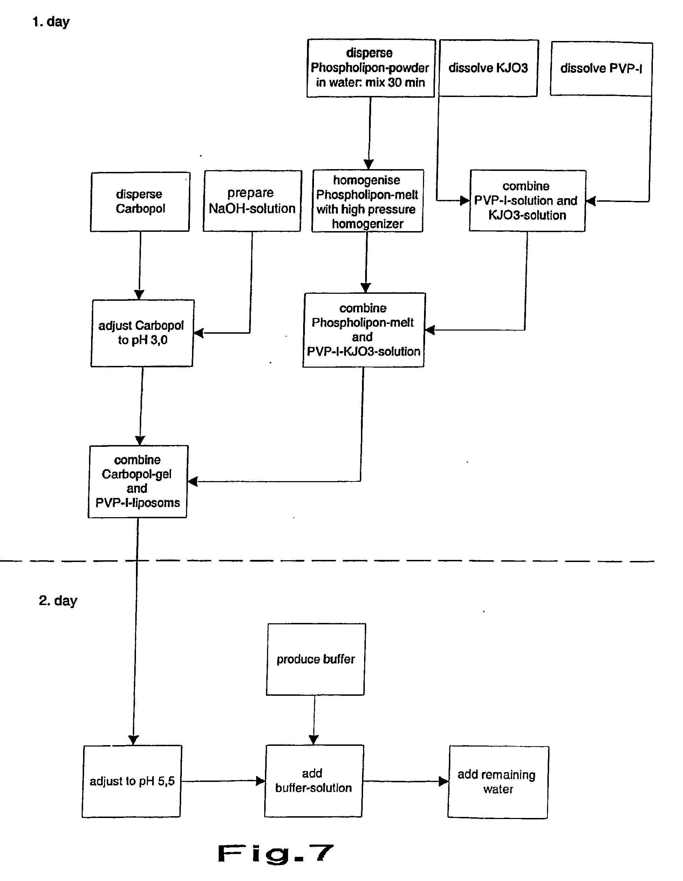

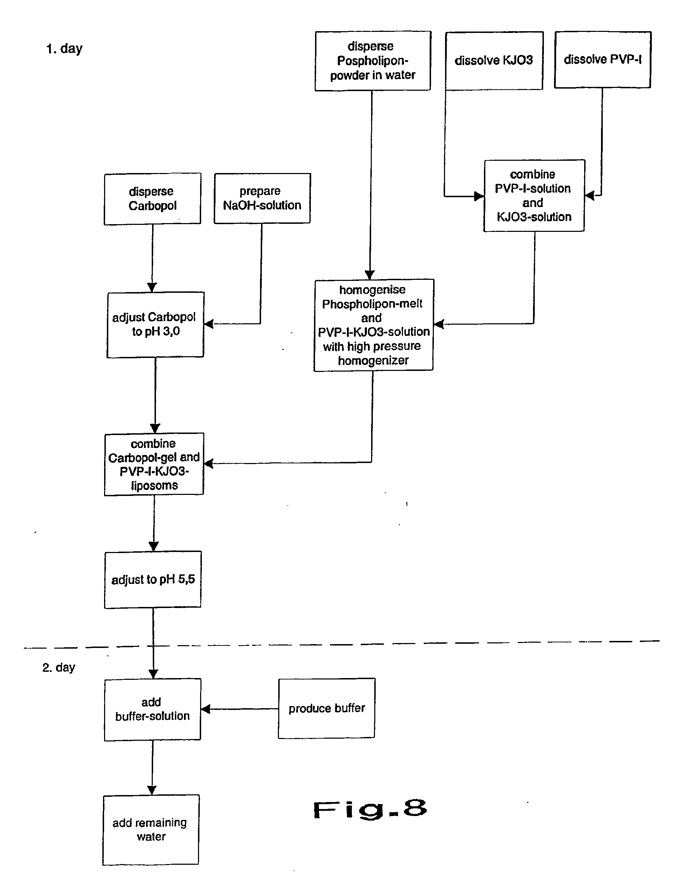

As already mentioned above, a particularly preferred embodiment of the invention is a hydrogel formulation comprising liposomes and PVP-iodine. Such hydrogel formulations usually comprise Phospholipon® 90 H (Aventis, Germany) as liposome-forming material and Carbopol® 980 NF (Noveon Inc., USA) as gel-forming substance. Phospholipon® 90 H is a 90% hydrogenated phosphatidylcholin preparation which is more storage-stable than unhydrogenated phosphatidylcholin preparations. Carbopol® is the trade name for Acrylic acid polymers that are commonly used for formation of pharmaceutically acceptable gels. The preferred hydrogel formulations may also comprise KIO₃, which serves for refurnishing iodine in the preparation. Citric acid and Na₂(HPO₄), which are used as buffering agents, advantageously influence the stability of the preparations. Flow charts of the production process are shown in Figures 1 to 8, with Figure 1 illustrating the currently preferred production process. A detailed discussion of this process is given in Example 6.

Examples are set out below that specifically illustrate the production of preferred embodiments of the invention. They are intended to illustrate how preparations according to the invention may be produced.

### Embodiment Example I - preparation for in vitro tests

In a 1000 ml glass flask, provided with glass beads for increased surface, 51.9 mg cholesterol and 213 mg hydrogenated soy bean lecithin were dissolved in a sufficient amount of a mixture of methanol and chloroform in a 2:1 ratio. The solvent was then evaporated under vacuum until a film was formed on the inner surface of the flask and on the glass beads.

2.4 g PVP iodine (containing about 10 % available iodine) were separately dissolved in 12 ml water.

Again in a separate vessel, 8.77 g sodium chloride and 1.78 g Na₂HPO₄·2H₂O were dissolved in 400 ml water. Further water was added up to a total volume of 980 ml, and then, approximately 12 ml 1N hydrochloric acid were added to adjust pH to 7.4. This solution was then topped up with water to exactly 1000 ml.

In a fourth vessel, 900 mg saccharose and 57 mg disodium succinate were dissolved in 12 ml water.

The PVP iodine solution was then added to the lipid film in the flask and the mixture was shaken until the film dissolved. The resulting liposome formulation was separated from the hydrated lipids in the flask. The product was centrifuged and the supernatant liquid was discarded. The saccharose solution was added ad 12 ml and the product was again centrifuged. Afterwards the supernatant liquid was again discarded. At this stage, a further washing step, using the saccharose solution or the sodium chloride buffer solution could be carried out.

After the last centrifugation step and discarding of the supernatant, 12 ml sodium chloride buffer solution was added, and the liposomes were homogenously distributed therein. The product was then distributed into vials each containing 2 ml liposome dispersion, and the vials were then subjected to a freeze-drying step.

After the freeze-drying, each vial comprised about 40 mg solids.

The method of Embodiment Example I has a minor disadvantage in that the PVP iodine solution used, due to the high percentage of solids, is rather viscous and thus more difficult to handle.

### Embodiment Example II

In a 2000 ml flask provided with glass beads to increase surface, 173 mg hydrogenated soy bean lecithin and 90 mg disodium succinate were dissolved in approximately 60 ml of a methanol/chloroform mix in a 2:1 ratio. The solvent was removed under vacuum until a film was formed.

4 g PVP iodine (10 % available iodine) were dissolved in 40 ml of the sodium chloride buffer solution described in Embodiment Example I, and were added to the lipid film in the flask. The flask was then shaken until the film dissolved and liposomes were formed.

The product was centrifuged and the supernatant liquid was discarded.

To the thus produced liposome pellet, further 40 ml sodium chloride buffer solution was added, and the centrifuging step was repeated. The supernatant was again discarded. At this stage, the washing step could be repeated where necessary.

After the final centrifuging and decanting step, 40 ml sodium chloride buffer solution was again added to the precipitated liposomes. The homogenous dispersion was then distributed into vials, each vial containing about 2 ml liposome dispersion, and the vials were then subjected to a freeze-drying step. This produced approximately 200 mg freeze-dried solids per vial.

From the freeze-dried solids of Examples I and II, further preparations were made as described in subsequent Embodiment Examples and Test Reports.

Like that of Embodiment Example I, the above-described method uses a hydrating step after film formation in the presence of organic solvents and aims at inclusion rates of 5 to 15 %. These methods generally produce rather large and often multilamellar liposomes.

The above-described methods can be modified by a high pressure filtering step through a suitable membrane such as a polycarbonate membrane after the raw liposomes have been formed or after any of the subsequent washing steps or directly by using high pressure homogenization. This produces much smaller, unilamellar liposomes at increased amounts of encapsulated agent.

Instead of high pressure homogenization, other prior art methods known to provide small uniform sized liposomes can be employed.

### Embodiment Example III

A hydrophilic (O/W) cream was prepared from 10 g hydrogenated soy bean lecithine/PVP iodine liposomes as described in Embodiment Example II; these were mixed with 4 g Polysorbate 40 (TM), 8 g cetylstearyl alcohol, 8 g glycerol, 24 g white vaseline, and water ad 100 g.

### Embodiment Example IV

An amphiphilic cream was prepared from 10 g hydrogenated soy bean lecithine/povidone iodine liposomes as described in Embodiment Example II; 7.5 g medium chain length tryglyceride, 7 g polyoxyethyleneglycerol monostearate, 6 g cetylstearyl alcohol, 8 g propylene glycol, 25 g white vaseline, and water ad 100 g.

### Embodiment Example V

A hydrophilic ointment which can be rinsed off with water was prepared using 10 g of liposomal PVP iodine as described in Embodiment Example II, 55 g Macrogol 400 (TM), 25 g Macrogol 4000 (TM), and water ad 100 g.

### Embodiment Example VI

A hydrogel was prepared from 4 g liposomal PVP iodine as described in Embodiment Example II, 0.5 g Carbopol® 980 NF (TM), sodium hydroxide ad pH 7.0, water ad 100 g. Further modifications of the above-described embodiments are envisaged.

Thus, the creams of Embodiment Examples IV and V can have an additional content of an agent known to promote the healing of wounds, such as allantoin.

Such an agent will be added in a pharmaceutically useful concentration, in the case of allantoin in the range of 0.1 to 0.5 g, per 100 g of cream. The wound healing agent can be incorporated in the cream base, in which case it will largely be outside the liposomes. It can, however, be partly or mostly incorporated in the liposomes, in which case it will be added at a corresponding suitable stage of the liposome preparation method.

Similar alternatives are easily envisaged on the basis of the further Embodiment Examples.

It is also possible to prepare embodiments similar to the above described ones, which comprise an agent capable of promoting the healing of wounds instead of, and not in addition to, the antiseptic agent as e. g. povidone iodine disclosed in the above Embodiment Examples. Presently, it is however preferred to use a wound healing promoting agent (if at all) in addition to an antiseptic agent.

For application of the inventive preparations to a patient, known systems can be used, such as pneumatic pump applicators, two-chamber gas pressure packs, aerosol spray dispensers etc.

In a pneumatic pump applicator, a bellows device is provided between an upstream and a downstream valve, both valves operating one way in the same direction. A supply of pharmaceutical preparation, such as an ointment or gel, is contained in a reservoir upstream of the valves-and-bellows device.

When compressing the bellows, the downstream valve opens and permits a dosed amount of preparation to leave the device for application. When the bellows is extended, this valve shuts and prevents reentry of the preparation. At the same time, the upstream valve opens and permits preparation from the reservoir to enter into the bellows, for release through the downstream valve upon the next compression step of the bellows.

The reservoir is sealed by a closure element which can move through the reservoir like a piston moves in a cylinder. By the stepwise emptying of the reservoir, this closure element is sucked into the reservoir, so that the remaining amount of pharmaceutical preparation in the reservoir is always sealed off, while at the same time the reservoir can be emptied. Such a device is useful for pasty preparations, creams, ointments etc.

In a two-chamber gas pressure pack, the pharmaceutical preparation is contained in a bag of flexible plastics film material. Often, this is high pressure polyethylene.

The bag is contained inside a gas tight pressure vessel which further contains a supply of pressurizing gas, very often a compressed inert gas like nitrogen or air.

The plastic film bag has only one outlet, which is gas-tightly connected to the interior wall of the pressure vessel, surrounding a single opening thereof. The pressurized gas in the vessel tends to compress the bag, driving the pharmaceutical preparation inside the bag out through the opening of the bag and thus through the opening of the vessel. A valve and, in case, spray-head device is provided in the vessel mouth. Operating the valve releases a spray mist, a jet of liquid or a portion of flowable solid such as cream. Using such a system, solutions, emulsions, creams, oitments and gels can be dosed and applied.

### Embodiment example VII

A Hydrogel was formulated according to the flow chart shown in figure 1. The amounts shown in Table 1 were used either for analytical or scale up preparations.

**Table I**

| Pos. | Substance | Amount (g/100g) | Scale up (kg/1500kg) |
|---|---|---|---|
| A | H₂O | 15,0 | 200,0 |
| A | Phospolipon 90 H | 3,0 | 45,0 |
| B | H₂O | 40,0 | 600,0 |
| B | Carbopol® 980 NF | 1,5 | 22,5 |
| C | H₂O | 2,0 | 30,0 |
| C | KIO₃ | 0,0708 | 1,09 |
| D | H₂O | 20,0 | 300,0 |
| D | PVP-iodine 30/06 Avalaible iodine (10%) | 3,0 | 45,0 |
| E | H₂O | 2,5 | 50,0 |
| F | H₂O | 2,5 | 50,0 |
| G | H₂O | 4,6 | 69,0 |
| G | NaOH solid | 0,46 | 6,9 |
| I | Citric acid, H₂O free | 0,1065 | 1,059 |
| I | Na₂(HPO)₄, H₂O free | 0,225 | 3,37 |
| I | H₂O | 3,0 | 45,0 |
| H | H₂O | ad 100,0 | ad 1500 |

Pos. stands for Position (see also below Table 2). Phospholipon® 90 H was purchased from Aventis (Germany). Carbopol® 980 NF was purchased from (Noveon Inc., USA) and PVP Iodine 30/06 was purchased from BASF (Germany).

In Table 2, column 2 the exact order of steps and the parameters of each step are given (see also Figure 1). Column 3 discusses non-exclusive alternatives. All steps were performed at room temperature except where indicated otherwise. All substances were of a purity grade common for pharmaceutical preparations such as described in the British Pharamcopeia.

**Table II**

| No. | Embodiment example VII | Alternatives |
|---|---|---|
| 1 | Carbopol 980 NF is mixed into H₂O without agglomeration (Pos. B). Stirring for 30 min at approx. 30 upm (units per minute) in conventional stirrer. Visual control for Polyacrylic acid-agglomerates. If necessary, homogenize gel in conventional homogenisator for 2 min at 3000 upm. | Substances: Other gel-forming substances may be used. Homogenization time can vary: shorten to 1min prolong to 10 min (caution! gel structure may be destroyed) |
| | | |
| | Subsequently stir gel for 30 min at 30 upm in conventional stirrer. Eventually control again for Polayacrylicacid-agglomerates. | Stirring time can be altered as desired. Only condition is that gel is free of agglomerates at the end. |
| | | |
| | If present, remove them and stir again for 15 min at 30 upm. Eventually homogenize again. | Swelling time may be altered from 15 min to 5 days. Preferably the gel has formed before other substances are added. |
| | | |
| | Let gel swell for at least 14 h. | Adjustment of pH to 2-8 may be performed at this stage. Adjustment to pH 3-6 is preferred. |
| 2 | Dissolve H₂O and KIO₃ completely in a suitable vessel (Pos. C). A 30-40% KIO₃-solution may also be used. | H₂O-temperature may be adjusted to anywhere between ambient temperature and 100 °C. |
| | | |
| | | KIO₃ is not obligatory. |
| 3 | Dissolve NaOH completely in H₂O (Pos. G). | NaOH is used in concentrations common for pharmaceutical preparations. Other Bases or substances suggested by the supplier of the gel forming substances may also be used for formation of gel structure as e.g. KOH, Triethanol-amine, 2-Amino-2-methyl-1-propanol, tris(hydroxymethyl)aminoethan, 2-hydroxypropyl-ethylen-diamine, diisopropanolamine. |
| 4 | Mix PVP-iodine into H₂O while stirring at 1000 upm in conventional stirrer (Pos. D). | Stirring time and speed can be altered arbitrarily. |
| | | |
| | Stir mixture for futher 60-70 min at 1000 upm until it is completely dissolved. | Important: PVP-Iodine has to be dissolved completely. |
| 5 | Warm H₂O to 65 °C while stirring with 1000 upm in conventional stirrer. Then add slowly Phospholipon® 90 H (Pos. A). Take care that no agglomerates are formed. | Possible temperature range: 40 °C - 120 °C. 50 °C - 75 °C is preferred because of phase transition temperature. |
| | | |
| | Stir dispersion for further 90 min at 65 °C - 70 °C and 1000 upm. | Other liposome-forming materials or mixtures thereof may be used. |
| | | |
| | Subsequently cool liposomal dispersion to ≤30 °C while stirring at 500 upm | Stirring time and speed: Is dependent on equipment. A complete dispersion has to be achieved. Apparatus of the rotor/stator principle, high pressure homogenisators, ultrasound or extrusion technology may also be used for stirring. |
| 6 | By adding the NaOH-solution (No. 3) the gel is adjusted to a pH of 3.0 (± 0,2). | Further processing to a gel may be feasible without pH pre-adjustment and is dependent on the gel-forming substance |
| 7 | The KIO₃ solution (No.2) is added to the PVP-Iodine solution (No. 4) while stirring at 1000 upm. Stirring continued for at least 60 min. | Reaction between KIO₃ and PVP-iodine is time dependent. To ensure a complete reaction, the stirring time has to adapted accordingly. Thus, stirring time may be between 10 min and 2 h |
| 8 | The PVP-iodine- KIO₃ -solution is pumped into the liposomal dispersion (No. 5). Subsequently it is stirred for 30 min at 1000 upm. | Stirring time is variable depending on until when an homogeneous mixture has formed. |
| 9 | The PVP-iodine- KIO₃-liposomes-dispesion is added to the gel (No. 6). | Stirring time is variable depending on until when an homogeneous mixture has formed. |
| | | |
| | It is stirred for 30 min at 30 upm. | Stirring time should be as short as possible so that gel structure gets not disrupted. |
| | | |
| | ***Subsequently homogenization is performed by forced circulation pumping for 2 min at 2800 upm.*** | |
| | | |
| | After checking for agglomerates, it may be homogenized for further 1 - 2 min. | |
| 10 | Remove agglomerates if present. | Adjust stirring time and speed to gel quality. |
| | | |
| | Add 50,0 kg NaOH-solution(in the scale up, point 3) while stirring at 30 upm. | Amounts of NaOH may vary. Adding of base by step wise adjustment until desired pH is achieved. |
| | | |
| | Stir for further 30 min at 30 upm at ≤30 °C. Cool if necessary. | |
| | | |
| | Determine pH and add additional NaOH until an pH of 5.5 (±0.2) is achieved. After each adding step stir for 20 min. | |
| | | |
| | After each adding step homogenize by circulation pressure pumping for 15 sec at 1000 upm. | |
| | | |
| | After adjustment of pH stir for further 15 min at 30 upm. Check pH and correct if necessary. | |
| | | |
| | After successful pH adjustment add remaining H₂O amount which depends on the amount of NaOH used. | |
| 11 | Mix buffer solution at 30 °C while stirring until it is completely dissolved (Pos. I). | Temperature can be raised to 40 °C. Other suitable buffers may also be used. |
| 12 | Buffer solution is added to the product (No. 10) while stirring for 15 min at 30 upm. | The desired product quality (storage stability) is achieved by addition of the buffer. |
| | | |
| | Degas by application of vacuum. | Stirring time is variable depending on until when an homogeneous mixture has formed. |
| | | |
| | | Degasing may be achieved by other means than vacuum. |
| 13 | Add the remaining H₂O-amount (Pos. H) and stir for 30 min at 25 upm | Stirring time is variable depending on until when an homogeneous mixture has formed. |
| | | |
| | Optionally homogenization may be performed by circulation pressure pumping for 15 sec at 1000 upm. | |
| | | |
| | Stir for another 30 min. | |
| | | |
| | Check visually for agglomerates | |

Positions E and F of Table I are used for washing the KIO₃- and PVP-iodine vessels (points 2 and 4 of Table II).

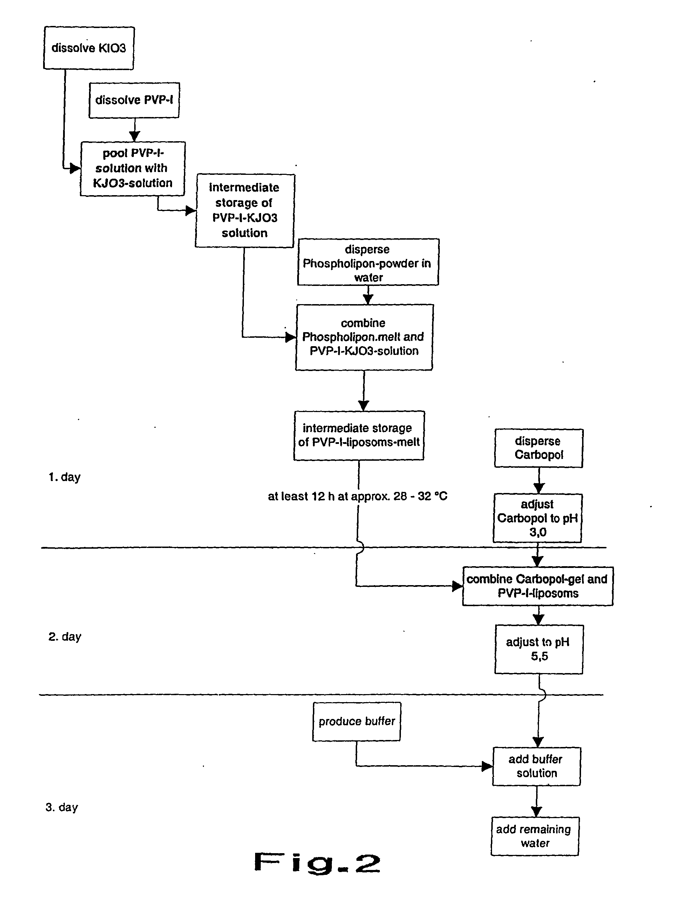

As mentioned above, the Hydrogel-Formulation is produced according to the method set out in Table 2 and Figure 1. Alternative methods become obvious from the flow charts of figures 2 to 8. The individual steps may be performed as set out above.

Using inventive preparations efficiency tests were then carried out, as follows:

### Test I

This was an in-vitro-test of the bactericidal effect provided by an inventive povidone iodine liposome preparation. The test was based on the quantitative suspension test as described in "Richtlinien der Deutschen Gesellschaft für Hygiene und Mikrobiologie", 1989. In this test, the bactericidal agent is used to kill staphylococcus aureus (ATCC 29213), a major problem in hospital hygiene.

The liposome preparation used was that of Embodiment Example I. At different contact times between 1 und 120 minutes, the minimum concentration of the preparation in water was determined which was capable of killing the staphilococci.

The results are shown in Table 3.

**TABLE III**

| Contact Time (Minutes) | Bactericidal Concentration |
|---|---|
| 1,2,3,4 | ≥ 0.060 % |
| 5,30,60 | ≥ 0.015 % |
| 120 | ≥ 0.007 % |

The results show that at short contact times (between 1 and 4 minutes) the bactericidal concentration is as low as 0.06 % and that at long contact times (120 minutes) the bactericidal concentration can be as low as 0.007 %.

### Test II

The virucidal and chlamydicidal activity of liposomal PVP-iodine has been studied, in cell cultures, by Wutzler et al., 9th European Congress for Clinical Microbiology and Infection Diseases, Berlin, March 1999. In cell cultures, liposomal PVP-iodine is highly effective against herpes simplex virus type 1 and adenovirus tpye 8, while the long-term cytotoxicity experiments indicated that the liposomal form is better tolerated than aqueous PVP-iodine by the majority of cell lines tested. PVP-iodine in liposomal form is not genotoxic.

### Test III

A 3% PVP-iodine hydrogel liposomal preparation was compared with a 3% PVP-iodine ointment, where the active agent was not in liposomal form. The agent was applied to standardized in vitro cultures of rat skin and peritoneal explants, as a screening for tissue compatibility of skin and wound antiinfectives.

The growth rate of the cultured explants was studied after 30 minutes exposure and incubation with a test substance.

Again, the substantially better toleration of the liposomal preparation was clearly shown in the results, in terms of peritoneum growth rate and skin growth rate.

With the ointment, the peritoneum growth rate reached 85%, and the skin growth rate reached 90%; with the liposomal hydrogel formulation, the peritoneum growth rate was 96%, and the skin growth rate was 108%; these values are to be compared with 100% values in a control test using Ringer's solution as the agent.

### Test IV

The toleration of liposomal PVP-iodine solutions for nasal applications was studied by investigating the influence of different test substances on ciliated epithelium cells, the most sensible cells of the mucous membrane. A cytotoxic damage of these cells which would cause a restriction of the mucociliar clearance can be determined by a detectable decrease of the ciliary vibration.

Human ciliated epithelium cells were analysed by an in-vitro method which enables the determination of the ciliary activity or ciliary vibration. The corresponding cells were exposed and incubated with 100 je. 1 test substance at a temperature of 37 C. After an incubation period of 5 minutes the ciliary vibration was measured.

By using this in-vitro method a nutriant solution (Dulbeco) as standard, a 0.2% chlorohexidine solution (typical antiseptic agent), conventional polyvidone iodine solutions (Betaisodona) of different concentrations (5.0%, 2.5% and 1.25% PVP-iodine) and a liposomal solution containing 4.5% of PVP-iodine were tested.

The substantially better toleration of the liposomal preparation was clearly shown in the results: if the ciliated epithelium cells were exposed to the Betaisodona solutions containing 5.0% or 2.5% PVP-iodine, no ciliary activity could be observed after the incubation period. Treating the cells with a chlorohexidine solution led to a decrease of the measured ciliary vibration in comparison to the standard (nutriant solution).

The low concentrated Betaisodona solution containing 1.25% PVP-iodine, didn't cause a detectable decrease of the ciliary activity. With respect to the measured ciliary vibration no differences to the standard (nutrian solution) could be determined by exposing the human ciliated epithelium cells to the concentrated liposomal 4.5% PVP-iodine solution.

These results indicate that the liposomal formulation is well tolerated for nasal application and advantageous with respect to for e. g. chlorohexidine or conventional Betaisodona® solutions.

## Claims

1. Use of iodine or at least one iodine complex, which contains the iodine in elementary form, for the manufacture of a pharmaceutical preparation for the treatment of atopic dermatitis,
**characterized in that** the preparation comprises said iodine or said at least one iodine complex in a pharmaceutically effective amount combined with pharmaceutically acceptable liposomes.

2. Use according to claim 1,
**characterized in that** the at least one iodine complex is PVP-iodine.

3. Use according to any of claims 1 or 2,
**characterized in that** the preparation comprises additionally wound-healing promoting agents, that promote granulation and epithel-formation such as dexpanthenol, allantoines, azulenes, tannins, vitamins, preferably vitamin B and derivatives thereof.

4. Use according to one of the preceding claims,
**characterized in that** the liposomes have a size in a range between 1 nm and 100 µm, preferably between 1 µm and 50 µm and most preferably between 1 µm and 25 µm.

5. Use according to one of the preceding claims,
**characterized in that** the liposomes release the active compound(s) over an extended time period, preferably over a time period of several hours duration.

6. Use according to one of the preceding claims,
**characterized in that** liposomes release the active compound(s) at the same release rate over the time of the release.

7. Use according to one of the preceding claims,
**characterized in that** the preparation comprises additives and adjuvants such as conserving agents, antioxidants and consistency-forming additives.

8. Use according to one of the preceding claims,
wherein the preparation is provided in the form of a solution, suspension, dispersion, ointment, spray, lotion, cream, gel or hydrogel comprising the iodine- or iodine complex-loaded liposomes.

9. Use according to one of the preceding claims,
wherein the preparation is provided in the form of a pharmaceutical solution-, suspension-, dispersion-, ointment-, lotion-, cream-, gel- or hydrogel-formulation that comprises:
• liposomes comprising a pharmaceutically acceptable liposomal membrane forming substance and
• a 0,1% to 5% by weight PVP-iodine solution,
wherein the liposomes are of a size with diameters between 1 µm and 50 µm and, in case, the formulation additionally comprises customary additives, adjuvants and auxiliary substances of a pharmaceutical solution-, suspension-, dispersion-, ointment-, lotion-, cream-, gel- or hydrogel-formulation.

10. Use according to claim 9,
**characterized in that** the liposomes are of a size with diameters between 1 µm and 25 µm.

11. Use according to one of the preceding claims,
wherein the preparation is preferably suitable for the treatment of various forms of atopic dermatitis including Eczema flexurarum, Eczema mulluscatum, Eczema verrucatum, Eczema vaccinatum, Eczema dyscoides, dyshidrotic eczema, microbial eczema, nummular eczema, seborrhoic eczema, perioral and periorbital dermatitis.

12. Use according to claim 11,
wherein the preparation is suitable for the treatment of the bacterial and viral secondary infections that occur in the course of atopic dermatitis, preferably for the treatment of *Staphylococcus aureus* infections and pyodermas such as *Impetigo contagiosa* and its derivatives and/or *Follicularis barbae.*

13. Use according to one of the preceding claims,
wherein the preparation can be used for the topic treatment of the various forms of atopic dermatitis, preferably in the face, on the neck, on the breast and back areas and on the extremities, more preferably at the joint flexures or the opposite sites thereof.

## Patentansprüche

1. Die Verwendung von Iod oder mindestens einem Iodkomplex, der Iod in elementarer Form enthält, für die Herstellung einer pharmazeutischen Zubereitung für die Behandlung von atopischer Dermatitis,
**dadurch gekennzeichnet, dass** die Zubereitung das genannte Iod oder den genannten mindestens einen Iodkomplex in einer pharmazeutisch aktiven Menge zusammen mit pharmazeutisch akzeptablen Liposomen enthält.

2. Die Verwendung gemäß Anspruch 1,
**dadurch gekennzeichnet, dass** der mindestens eine Iodkomplex PVP-Iod ist.

3. Die Verwendung gemäß einem der Ansprüche 1 oder 2,
**dadurch gekennzeichnet, dass** die Zubereitung zusätzlich wundheilungsfördernde Mittel enthält, die die Granulation- und die Bildung von Epithelgewebe fördern wie Dexpanthenol, Allantoine, Azulene, Tannine, Vitamine, bevorzugt Vitamin B und Derivate hiervon.

4. Die Verwendung gemäß einem der vorhergehenden Ansprüche,
**dadurch gekennzeichnet, dass** die Liposomen eine Größe im Bereich zwischen 1 nm und 100 µm, bevorzugt zwischen 1 µm und 50 µm und am meisten bevorzugt zwischen 1 µm und 25 µm aufweisen.

5. Die Verwendung gemäß einem der vorhergehenden Ansprüche,
**dadurch gekennzeichnet, dass** die Liposomen den Wirkstoff/die Wirkstoffe über eine längere Zeitdauer, bevorzugt über eine Zeitdauer von mehreren Stunden Länge freisetzen.

6. Die Verwendung gemäß einem der vorhergehenden Ansprüche,
**dadurch gekennzeichnet, dass** die Liposomen den Wirkstoff/die Wirkstoffe mit der gleichen Freisetzungsrate über die Dauer der Freisetzung freisetzen.

7. Die Verwendung gemäß einem der vorhergehenden Ansprüche,
**dadurch gekennzeichnet, dass** die Zubereitung Additive und Hilfsstoffe wie Konservierungsstoffe, Antioxidantien und konsistenzgebende Additive beinhaltet.

8. Die Verwendung gemäß einem der vorhergehenden Ansprüche,
wobei die Zubereitung in Form einer Lösung, einer Suspension, einer Dispersion, einer Salbe, eines Sprays, einer Lotion, einer Creme, eines Gels oder eines Hydrogels beinhaltend die Iod- oder Iodkomplex-beladenen Liposomen bereitgestellt wird.

9. Die Verwendung gemäß einem der vorhergehenden Ansprüche,
wobei die Zubereitung in Form einer pharmazeutischen Lösungs-, Suspensions-, Dispersions-, Salben-, Lotions-, Creme-, Gel- oder Hydrogelformulierung bereitgestellt wird, umfassend:
• Liposomen beinhaltend eine pharmazeutisch akzeptable liposomale membranbildende Substanz und
• eine Lösung mit 0,1 Gew.-% bis 5 Gew.-% PVP-Iod,
wobei die Liposomengrößen mit ihrem Durchmesser zwischen 1 µm und 50 µm liegen und die Formulierung zusätzlich herkömmliche Additive, Hilfsstoffe und Hilfsstoffe einer pharmazeutischen Lösungs-, Suspensions-, Dispersions-, Salben-, Lotions-, Creme-, Gel- oder Hydrogelformulierung enthält.

10. Die Verwendung gemäß Anspruch 9,
**dadurch gekennzeichnet, dass** die Liposomengrößen mit ihrem Durchmesser zwischen 1 µm und 25 µm liegen.

11. Die Verwendung gemäß einem der vorhergehenden Ansprüche,
wobei die Zubereitung besonders für die Behandlung von verschiedenen Formen der atopischen Dermatitis geeignet ist, einschließlich Ekzema flexurarum, Ekzema mulluscatum, Ekzema verrucatum, Ekzema vaccinatum, Ekzema dyscoides, dyshidrotische Ekzeme, mikrobielle Ekzeme, dysregulativ-mikrobielle Ekzeme, seborrhoische Ekzeme, periorale und periorbitale Dermatitis.

12. Die Verwendung gemäß Anspruch 11,
wobei die Zubereitung für die Behandlung von bakteriellen und viralen Sekundärinfektionen geeignet ist, die im Verlaufe von atopischer Dermatitis auftreten, bevorzugt für die Behandlung von *Staphylococcus aureus* Infektionen und Pyodermien wie *Impetigo contagiosa* und seinen Derivaten und/oder *Follicularis barbae.*

13. Die Verwendung gemäß einem der vorhergehenden Ansprüche,
wobei die Zubereitung für die topische Behandlung von verschiedenen Formen der atopischen Dermatitis verwendet werden kann, bevorzugt im Gesicht, am Hals, auf der Brust und im Rückenbereich und an den Extremitäten, bevorzugter an den Gelenkbeugen oder den gegenüberliegenden Bereichen.

## Revendications

1. Utilisation d'iode ou d'au moins un complexe d'iode, qui contient l'iode sous sa forme élémentaire, pour la fabrication d'une préparation pharmaceutique destinée au traitement de la dermatite atopique,
**caractérisée en ce que** la préparation comprend ledit iode ou ledit ou lesdits complexes d'iode en une quantité pharmaceutiquement efficace combinés à des liposomes pharmaceutiquement acceptables.

2. Utilisation selon la revendication 1,
**caractérisée en ce que** le ou les complexes d'iode sont un ou des complexes de PVP-iode.

3. Utilisation selon l'une quelconque des revendications 1 ou 2,
**caractérisée en ce que** la préparation comprend en outre des agents favorisant la cicatrisation, qui favorisent la granulation et la formation d'épithélium, tels que le dexpanthénol, les allantoïnes, les azulènes, les tannins, les vitamines, de préférence la vitamine B et ses dérivés.

4. Utilisation selon l'une quelconque des revendications précédentes,
**caractérisée en ce que** les liposomes ont une taille dans une plage comprise entre 1 nm et 100 µm, de préférence entre 1 µm et 50 µm et idéalement entre 1 µm et 25 µm.

5. Utilisation selon l'une quelconque des revendications précédentes,
**caractérisée en ce que** les liposomes libèrent le(s) composé(s) actif(s) sur une période de temps prolongée, de préférence sur une période de temps de plusieurs heures.

6. Utilisation selon l'une quelconque des revendications précédentes,
**caractérisée en ce que** les liposomes libèrent le(s) composé(s) actif(s) à la même vitesse de libération pendant toute la durée de la libération.

7. Utilisation selon l'une quelconque des revendications précédentes,
**caractérisée en ce que** la préparation comprend des additifs et des adjuvants tels que des agents de conservation, des antioxydants, et des additifs de consistance.

8. Utilisation selon l'une quelconque des revendications précédentes,
dans laquelle la préparation est fournie sous la forme d'une solution, d'une suspension, d'une dispersion, d'un onguent, d'un spray, d'une lotion, d'une crème, d'un gel ou d'un hydrogel comprenant les liposomes chargés d'iode ou de complexe d'iode.

9. Utilisation selon l'une quelconque des revendications précédentes,
dans laquelle la préparation est fournie sous la forme d'une formulation de solution, de suspension, de dispersion, d'onguent, de lotion, de crème, de gel ou d'hydrogel, qui comprend :
- des liposomes comprenant une substance formant une membrane liposomale pharmaceutiquement acceptable et
- une solution de PVP-iode à 0,1 % à 5 % en poids,
les liposomes étant d'une taille avec des diamètres compris entre 1 µm et 50 µm et, éventuellement, la formulation comprend en outre des additifs, adjuvants et substances auxiliaires ordinaires pour une formulation de solution, de suspension, de dispersion, d'onguent, de lotion, de crème, de gel ou d'hydrogel.

10. Utilisation la revendication 9,
**caractérisée en ce que** les liposomes sont d'une taille avec des diamètres compris entre 1 µm et 25 µm.

11. Utilisation selon l'une quelconque des revendications précédentes,
dans laquelle la préparation est de préférence adaptée pour le traitement de différentes formes de dermatite atopique, comprenant l'Eczema flexurarum, l'Eczema molluscatum, l'Eczema verrucatum, l'Eczema vaccinatum, l'eczéma discoïde, l'eczéma dyshidrotique, l'eczéma microbien, l'eczéma nummulaire, l'eczéma séborrhéique, la dermatite périorale et périorbitaire.

12. Utilisation selon la revendication 11,
dans laquelle la préparation est adaptée pour le traitement des infections secondaires virales et bactériennes qui surviennent durant la dermatite atopique, de préférence pour le traitement des infections par *Staphylococcus aureus* et des pyodermites telles que l'*Impetigo contagiosa* et ses dérivés et/ou *Follicularis barbae.*

13. Utilisation selon l'une quelconque des revendications précédentes,
dans laquelle la préparation peut être utilisée pour le traitement topique des différentes formes de dermatite atopique, de préférence sur le visage, sur le coup, sur les régions de la poitrine et des épaules, et sur les extrémités, de préférence aux plis des articulations ou sur les sites opposés.
